# EUROPEAN PATENT APPLICATION

(11) **EP 4 389 204 A1**
(43) Date of publication of application: **26.06.2024**
(21) Application number: 22216369.3
(22) Date of filing: 23.12.2022
(51) Int. Cl.: A61N 5/10, G06T 7/00, A61B 6/00

(54) **A METHOD AND APPARATUS FOR PROCESSING MEDICAL IMAGES TO ASSIST RADIOTHERAPY PLANNING**

(71) Applicant: Koninklijke Philips N.V., 5656 AG Eindhoven (NL)
(72) Inventor: ANTO, Gipson Joe, Eindhoven (NL); PRASAD, Girish, 5656AG Eindhoven (NL); HEGDE, Shashank Sathyanarayana, 5656AG Eindhoven (NL); C, Arun, 5656AG Eindhoven (NL); NAGARAJ, Yeshwanth, 5656AG Eindhoven (NL)
(74) Representative: Philips Intellectual Property & Standards

(57) **Abstract**

The present invention relates to a system and method for assisting radiotherapy planning. A data storage is provided comprising a patient support device library, the patient support device library comprising patient support device data for a plurality of patient support devices. A source medical image of a patient on a scanning couch is analysed to determine a reference plane. A patient support device is selected from the patient support device library, to select patient support device data for the selected patient support device. A modified medical image, based on the source medical image, wherein generating the modified medical image comprises positioning a representation of the patient support device relative to the reference plane.

## Description

### FIELD OF THE INVENTION

The present invention is directed to a computer implemented method of processing medical images to assist radiotherapy planning, and to an apparatus for carrying out image analysis. In particular, the present invention is directed to a computer implemented method of processing synthetic computed tomography (sCT) images.

### BACKGROUND OF THE INVENTION

Historically computed tomography (CT) images have been the primary images relied on for radiation oncology treatment planning. When planning radiation treatment, it is important to accurately compute radiation dose. CT images provide electron density information (in the form of CT numbers) which is used for dose computation. Other imaging modalities, such as magnetic resonance imaging (MRI), positron emission tomography (PET), and single-photon emission computed tomography (SPECT), may additionally be used as secondary images. These imaging modalities are capable of obtaining images with good resolution and functional imaging advantages - for example, MRI scanning modality offers superior soft tissue contrast for target and organ at risk (OAR) delineation, and dynamic imaging techniques for motion management, without any radiation dose. However, because images obtained by MRI do not provide electron density information for dose computation, it is necessary to use a combination of images obtained by MRI and images obtained by CT. This imposes (at least) two scans on the patient, resulting in additional burden on patients and clinical personnel. Further, since MRI images are reconstructed images, it can be challenging to maintain geometric fidelity. Geometric fidelity may depend on e.g., uniformity of magnetic field and/or the reconstruction algorithm.

Synthetic CT images are CT equivalent images derived from non-CT scanning modalities and which include CT number information. MRI is the most widely used imaging modality to create synthetic CT images. Synthetic CT images can be derived from images obtained by MRI using an appropriate algorithm.

It would be desirable to facilitate highly accurate radiation dose calculation, whilst avoiding the need to carry out multiple scans on the patient.

### SUMMARY OF THE INVENTION

The invention is defined by the claims.

According to examples in accordance with an aspect of the invention, there is provided a system for assisting radiotherapy planning, the system comprising:
a data storage comprising a patient support device library, the patient support device library comprising patient support device data for a plurality of patient support devices; and
a processor configured to:
   receive a source medical image of a patient on a scanning couch;
   determine a reference plane based on the source medical image;
   select a patient support device from the patient support device library;
   receive patient support device data for the selected patient support device; and
   generate a modified medical image, based on the source medical image, wherein generating the modified medical image comprises positioning a representation of the selected patient support device relative to the reference plane.

In this way, a source medical image can be automatically modified to include a representation of a patient support device (e.g., treatment couch, head rest, base plate) that reflects the type of patient support device to be used during radiotherapy (as opposed to the scanning couch). This can help to facilitate radiotherapy planning. Further, because the patient support device can be selected from among a plurality of different possible treatment patient support devices, the system offers greater flexibility in treatment planning. That is, the system facilitates t radiotherapy planning using any of various different patient support devices, rather than being limited to a single "known" patient support device, offering users improved flexibility.

The source medical image may be an MRI image obtained by magnetic resonance imaging and the processor may be further configured to:
generate a synthetic computed tomography image based on the source medical image;
   and
generate the modified medical image by inserting a representation of the patient support device, at a location defined relative to the reference plane, into the synthetic computed tomography image. The system facilitates convenient radiotherapy planning by providing images that include electron density information, soft tissue contrast and patient support device information. Furthermore, the system facilitates obtaining these images without subjecting the patient to multiple scans conducted using different scanning modalities.

The patient support device data may comprise:
(i) physical dimensions of the patient support device; and
(ii) an electron density property of the patient support device and/or a physical density property of the patient support device.

By incorporating this information into the modified medical image, it is possible to take account of the presence of the patient support device during radiotherapy when planning treatment (i.e., it is possible to account for attenuation of radiation by the patient support device). In particular, incorporating this information in the modified medical image enables more accurate dose calculation, in a convenient way.

The patient support device data may comprise physical dimensions of the patient support device and an electron density property of the patient support device. The patient support device data may comprise physical dimensions of the patient support device and a physical density property of the patient support device.

The processor may be further configured to:
receive a patient support device selection input; and
select the patient support device based on the patient support device selection input, wherein the patient support device selection input indicates a radiotherapy system for carrying out a planned radiotherapy treatment.

In response to a user selecting the radiotherapy system to be used to carry out the planned radiotherapy, the system automatically selects the appropriate patient support device from the patient support device library. In this way, the system facilitates accurate and convenient radiotherapy planning that does not require the user to have an in-depth knowledge of the patient support device used in a radiotherapy system.

The source medical image may comprise a representation of a plurality of markers associated with the scanning couch and the processor may be configured to determine the reference plane by:
detecting the plurality of markers associated with the scanning couch;
calculating the position of the scanning couch in the source medical image with respect to an image reference frame based on the locations of the plurality of markers; and
determining a reference plane based on the position of the scanning couch in the source medical image.

By identifying the location of the markers in the source medical image, the system can determine the position of the scanning couch in the image. Further, based on that information, the system can compute the reference of the scanning couch. Identifying the reference in the source medical image, with respect to the image reference frame, enables the system to insert the patient support device (e.g., the treatment couch) at the appropriate location in the modified medical image.

The patient support device data may comprise information indicating a configuration of intensity markers characteristic of the patient support device and the processor may be configured to:
process the source medical image to identify the configuration of a plurality of intensity markers;
run a patient support device identification algorithm configured to identify a patient support device present in the source medical image based on the identified configuration of the plurality of intensity markers and the patient support device data; and
determine the position of the patient support device in the source medical image relative to the reference plane based on the configuration of a plurality of intensity markers.

The patient support device may comprise a treatment couch, a headrest and/or an accessory for fixing a headrest to a treatment couch.

The system may further comprise carrying out a dose calculation, based on the modified medical image.

By taking the presence of the treatment couch during radiotherapy into account, the system facilitates highly accurate dose calculation.

The source medical image may comprise a representation of a plurality of markers and the processor may be further configured to:
process the source medical image to obtain imaged configuration information indicating the configuration of the markers in the source medical image; and
run a geometric distortion algorithm, wherein the geometric distortion algorithm is configured to:
   compare the imaged configuration information to reference configuration information, wherein the reference configuration information indicates the physical configuration of markers; and
   calculate and output geometric distortion information, based on the comparison of the imaged configuration information and the reference configuration information.

The imaged configuration information may indicate the apparent distances between the markers based on the source medical image and the reference configuration information may indicate the physical distances between the markers.

The geometric distortion information may comprise a geometric distortion metric, wherein the geometric distortion metric indicates a measure of geometric distortion in the source medical image.

The source medical image may be an MRI image.

The method may further comprise analysing the geometric distortion information to determine if it is indicative of the level of geometric distortion in the source medical image being beyond an acceptable limit/tolerance level.

According to another aspect of the invention, there is provided a method of radiation treatment planning, the method comprising:
obtaining a source medical image, using magnetic resonance imaging, wherein obtaining the source medical image comprises imaging a patient on a scanning couch;
processing the source medical image to obtain a synthetic computed tomography image;
determining a reference plane of the synthetic computed tomography image, based on the source medical image;
selecting a patient support device from a patient support device library;
receiving patient support device data; and
generating a modified medical image by inserting a representation of the patient support device, at a location defined relative to the reference plane, into an image based on the source medical image.

The method may further comprise carrying out a dose calculation, based on the modified medical image.

According to another aspect of the invention, there is provided a computer implemented method of processing a medical image, comprising:
receiving a source medical image of a patient on a scanning couch;
processing the source medical image to determine a reference plane;
selecting a patient support device from a patient support device;
receiving patient support device data for the selected patient support device; and
generating a modified medical image, based on the source medical image, by inserting a representation of the patient support device, at a location defined relative to the reference plane, into the synthetic computed tomography image.

The computer implemented method may further comprise:
generating a synthetic computed tomography image based on the source medical image; and
generating the modified medical image by inserting a representation of the patient support device into the synthetic computed tomography image.

The computer implemented method may further comprise carrying out a dose calculation, based on the modified medical image.

The patient support device data may comprise information indicating a configuration of intensity markers characteristic of the patient support device and the method may further comprise:
identifying the configuration of a plurality of intensity markers present in the source medical image;
running a patient support device identification algorithm configured to identify a patient support device present in the source medical image based on the identified configuration of the plurality of intensity markers and the patient support device data; and
determining the position of the patient support device in the source medical image relative to the reference plane based on the configuration of a plurality of intensity markers.

The computer implemented method may further comprise:
processing the source medical image to obtain imaged configuration information indicating the configuration of the markers in the source medical image; and
running a geometric distortion algorithm, wherein the geometric distortion algorithm is configured to:
   compare the imaged configuration information to reference configuration information, wherein the reference configuration information indicates the physical configuration of markers; and
   calculate and output a geometric distortion metric, based on the comparison of the imaged configuration information and the reference configuration information, wherein the geometric distortion metric is a measure of geometric fidelity for the source medical image, wherein the source medical image comprises a representation of a plurality of markers.

The imaged configuration information may indicate the apparent distances between the markers based on the source medical image and the reference configuration information may indicate the physical distances between the markers.

According to another aspect of the invention, there is provided a computer-readable storage medium comprising instructions which, when executed by a computer, cause the computer to carry out the method described above.

According to a further aspect of the invention, there is provided a computer implemented method for determining geometric fidelity of a medical image, the method comprising:
receiving a source medical image, wherein the source medical image comprises a representation of a plurality of markers;
processing the source medical image to obtain imaged configuration information indicating the configuration of the markers in the source medical image; and
running a geometric distortion algorithm, wherein the geometric distortion algorithm is configured to:
   compare the imaged configuration information to reference configuration information, wherein the reference configuration information indicates the physical configuration of markers; and
   calculate and output geometric distortion information, based on the comparison of the imaged configuration information and the reference configuration information.

The geometric distortion information may be a geometric distortion metric that provides a measure of geometric fidelity for the source medical image.

The imaged configuration information may indicate the apparent distances between the markers based on the source medical image and the reference configuration information may indicate the physical distances between the markers.

The source medical image may be an MRI image.

The method may further comprise analysing the geometric distortion information (e.g., geometric distortion metric) to determine if it is indicative of the level of geometric distortion in the source medical image being beyond an acceptable limit/tolerance level. Analysing the geometric distortion metric may comprise comparing the geometric distortion metric to a geometric distortion threshold. The method may comprise outputting an alert to the user in response to determining that the geometric distortion information indicates that the level of geometric distortion in the source medical image is beyond an acceptable limit.

The method may comprise displaying the geometric distortion information.

The method may comprise displaying the alert.

According to a further aspect of the invention, there is provided a system comprising a processor configured to carry out the method for determining geometric fidelity of a medical image.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig 1 is a schematic diagram showing a system for assisting a user in planning radiotherapy;
Fig 2 is a flow diagram illustrating a computer-implemented method according to one or more embodiments of the invention;
Fig 3 shows example synthetic computed tomography images generated by a method according to embodiments of the invention;
Fig 4 is a flow diagram illustrating a computer-implemented method according to one or more embodiments of the invention;
Fig 5 is a flow diagram illustrating a computer-implemented method according to one or more embodiments of the invention;
Fig 6 is a schematic diagram illustrating examples of patient support devices;
Fig 7 illustrates a computer implemented method for determining geometric fidelity of a medical image; and
Fig 8 illustrates a patient support device marked with a plurality of MRI markers.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a system for assisting radiotherapy planning. The system comprises a data storage comprising a patient support device library, the patient support device library comprising patient support device data for a plurality of patient support devices. The system further comprises a processor configured to: receive a source medical image of a patient on a scanning couch, determine a reference plane based on the source medical image, select a patient support device from the patient support device library, receive patient support device data for the selected patient support device; and generate a modified medical image, based on the source medical image, wherein generating the modified medical image comprises positioning a representation of the patient support device relative to the reference plane.

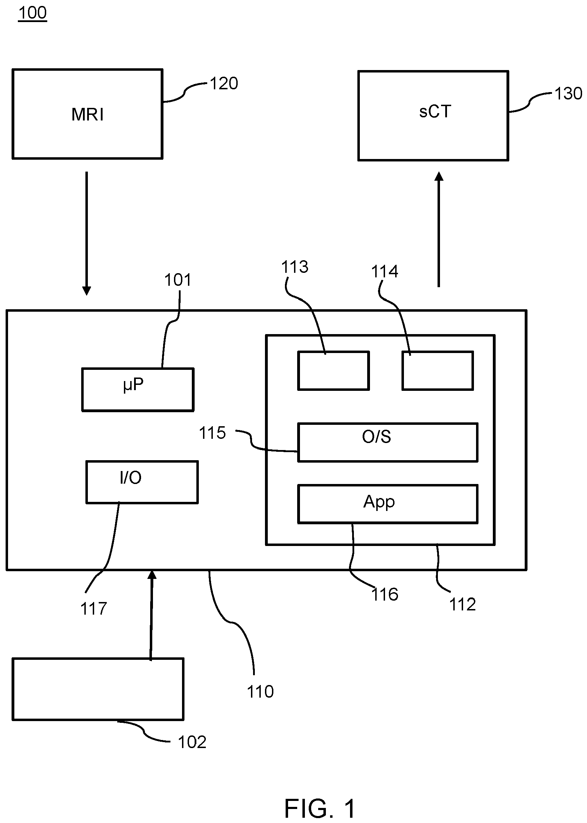

Fig 1 illustrates an embodiment of the invention, in which the system 100 comprises a computer 110 and a database 102 which comprises a patient support device library. The computer comprises a processor 111 and is communicatively coupled to the database 102 such that it can retrieve data from the patient support device library for processing by the processor 111. The computer 110 is configured to receive a source medical image (e.g., an MRI image), to process the MRI image to obtain an sCT image, and to insert a representation of a patient support device into the sCT image (thereby providing a modified medical image). The modified medical image is then output by the computer 110.

The patient support device library comprises information relating to a plurality of patient support devices. For each patient support device entry in the library, the database stores information relating to the physical dimensions and the attenuation characteristics (e.g., physical density, electron density - HU values) of the patient support device. This information can be stored as contours/images, for example.

By incorporating patient support device information into the images relied on to plan radiotherapy treatment, the system facilitates convenient and accurate treatment planning.

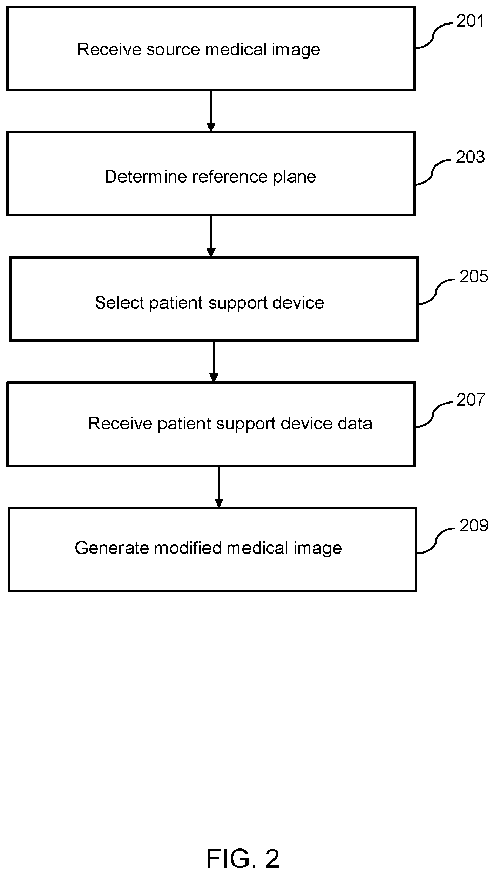

The system of Fig 1 can generate modified medical images, in which a representation of the patient support device is incorporated into the medical image, according to various different methods. Some example embodiments of such methods are described in relation to Figs 2 to 6.

Fig 1 illustrates an example of a computer 110 within which one or more parts of an embodiment may be employed. Various operations discussed herein may utilize the capabilities of the computer 110. In this regard, it is to be understood that system functional blocks can run on a single computer or may be distributed over several computers and locations (e.g., connected via internet).

The computer 110 includes, but is not limited to, PCs, workstations, laptops, PDAs, palm devices, servers, storages, and the like. Generally, in terms of hardware architecture, the computer 110 may include one or more processors 111, memory 112, and one or more I/O devices 117 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 111 is a hardware device for executing software that can be stored in the memory 112. The processor 111 can be virtually any custom made or commercially available processor, a central processing unit (CPU), a digital signal processor (DSP), or an auxiliary processor among several processors associated with the computer 110, and the processor 111 may be a semiconductor-based microprocessor (in the form of a microchip) or a microprocessor.

The memory 112 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 112 may incorporate electronic, magnetic, optical, and/or other types of storage media. Note that the memory 112 can have a distributed architecture, where various components are situated remote from one another, but can be accessed by the processor 111.

The software in the memory 112 may include one or more separate programs, each of which comprises an ordered listing of executable instructions for implementing logical functions. The software in the memory 112 includes a suitable operating system (O/S) 115, compiler 114, source code 113, and one or more applications 116 in accordance with exemplary embodiments. As illustrated, the application 116 comprises numerous functional components for implementing the features and operations of the exemplary embodiments. The application 116 of the computer 110 may represent various applications, computational units, logic, functional units, processes, operations, virtual entities, and/or modules in accordance with exemplary embodiments, but the application 116 is not meant to be a limitation.

The operating system 115 controls the execution of other computer programs, and provides scheduling, input-output control, file and data management, memory management, and communication control and related services. It is contemplated by the inventors that the application 116 for implementing exemplary embodiments may be applicable on all commercially available operating systems.

Application 116 may be a source program, executable program (object code), script, or any other entity comprising a set of instructions to be performed. When a source program, then the program is usually translated via a compiler (such as the compiler 114), assembler, interpreter, or the like, which may or may not be included within the memory 112, so as to operate properly in connection with the O/S 115. Furthermore, the application 116 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 117 may include input devices such as, for example but not limited to, a mouse, keyboard, scanner, microphone, camera, etc. Furthermore, the I/O devices 117 may also include output devices, for example but not limited to a printer, display, etc. Finally, the I/O devices 117 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 117 also include components for communicating over various networks, such as the Internet or intranet.

If the computer 110 is a PC, workstation, intelligent device or the like, the software in the memory 112 may further include a basic input output system (BIOS) (omitted for simplicity). The BIOS is a set of essential software routines that initialize and test hardware at startup, start the O/S 115, and support the transfer of data among the hardware devices. The BIOS is stored in some type of read-onlymemory, such as ROM, PROM, EPROM, EEPROM or the like, so that the BIOS can be executed when the computer 110 is activated.

When the computer 110 is in operation, the processor 111 is configured to execute software stored within the memory 112, to communicate data to and from the memory 112, and to generally control operations of the computer 110 pursuant to the software. The application 116 and the O/S 115 are read, in whole or in part, by the processor 111, perhaps buffered within the processor 111, and then executed.

When the application 116 is implemented in software it should be noted that the application 116 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 116 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Fig 2 illustrates an example embodiment of a computer implemented method (which can be implemented using a system of the type described in relation to Fig 1). In this embodiment, the source medical image is an MRI image.

The patient support device library comprises information relating to treatment couches (e.g., linear accelerator couches) on which a patient can be supported during radiotherapy treatment. A source medical image can be obtained of a patient while the patient is supported by a scanning couch. A processor receives 201 the source medical image and processes it, 203, to determine an image reference plane. For example, the image reference plane may be the plane of the scanning couch.

The position of the image reference plane can be determined by the user, or identified automatically. For example, the image reference plane can be determined automatically by analysing the image to determine the location of the image isocenter and calculating the position of the scanning couch relative to the image isocenter (to determine the plane of the scanning couch, and therefore the reference plane).

A treatment couch is selected, 205, from the patient support device library. The processor may be configured to select the treatment couch automatically (e.g., it may be configured to select a default treatment couch). Alternatively, the processor may be configured to select the treatment couch based on a user input (i.e., a user selection). For example, the user may input the treatment to be carried out on the patient. The processor may be configured to automatically select the appropriate treatment couch, based on the selected treatment.

The processor retrieves information, 207, (physical dimensions, attenuation characteristics) relating to the treatment couch from the database storing the patient support device library. Using this information, the processor generates, 209, a modified medical image, based on the source medical image.

The processor generates the modified medical image by converting the source medical image into a synthetic CT image and inserting a representation of the selected treatment couch into the synthetic CT image. The representation of the selected patient support device may be inserted at the image reference plane (the plane of the scanning couch), since the image reference plane of the synthetic CT image directly corresponds to the image reference plane of the MRI image serving as the source medical image

The representation of the selected patient support device incorporates the physical dimensions and attenuation information relating to the patient support device. Accordingly, the modified medical image can be used to plan radiotherapy in an improved way.

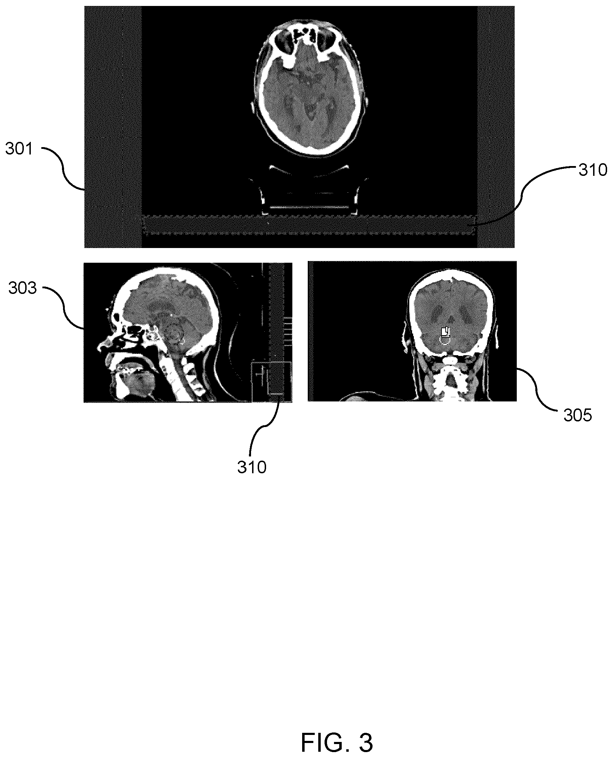

Fig 3 illustrates an example of a set of synthetic CT images 301, 303, 305. In the first image 301 and the second image 303, the image plane cuts through the patient support device. Accordingly, a treatment couch 310 has been inserted into the image. The third image 305 shows the coronal plane, which does not cut through the patient support device and therefore no patient support device is visible in the image.

In some embodiments, the position of the reference plane can be identified in the source medical image by providing the scanning couch with MRI markers. The MRI markers appear in the source medical image as bright areas and their locations can be determined by intensity detection. Accordingly, the source medical image can be processed to detect the plurality of markers associated with the scanning couch, in order to determine the reference plane of the source medical image (and the modified medical image).

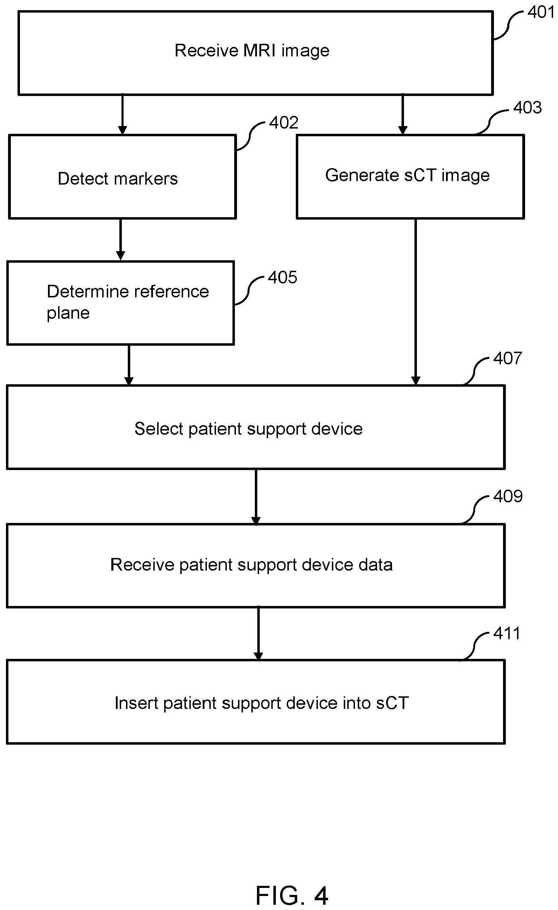

Fig 4 illustrates an example embodiment of a computer implemented method (which can be implemented using a system of the type described in relation to Fig 1), in which MRI markers are used to identify the position on the reference plane.

The computer receives, 401, the source medical image (which is an MRI image). The source medical image is processed, using intensity detection, to identify the positions of the MRI markers, 402, in the source medical image. This information is used to identify the position of the scanning couch, and thereby the position of the reference plane, 405, in the source medical image. For example, the MRI image may be processed calculate the position of the scanning couch in the source medical image with respect to an image reference frame (e.g., DICOM) based on the locations of the plurality of markers. Using this information, the processor may be configured to determine the plane of the scanning couch in DICOM co-ordinates.

Further, the computer generates, 403, an sCT image based on the source medical image.

A treatment couch is selected, 407, from a treatment couch library (automatically, or based on user input). Treatment couch data (e.g., a representation containing the physical dimensions and attenuation properties of the treatment couch) is retrieved, 409, from the treatment couch library.

Lastly, the selected representation of the treatment couch is inserted, 411, into the sCT image at the reference plane.

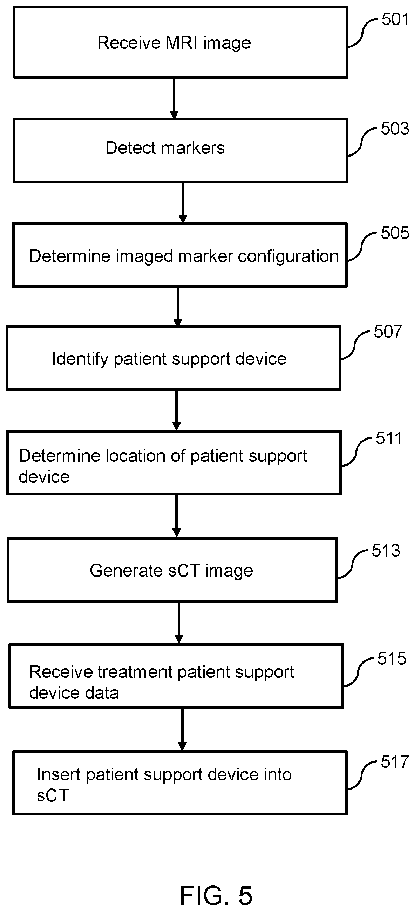

Fig 5 illustrates an example embodiment of the invention, in which MRI markers are used to automatically select the appropriate patient support device to be inserted into the modified medical image.

In some embodiments, the patient support device on which the patient is scanned to obtain the source medical image may be provided with MRI markers. Accordingly, the source medical image received, 501, by the computer provides information about the configuration of the MRI markers on the patient support device.

The computer processes the source medical image to detect, 503, the MRI markers using intensity detection. In this way, the computer can analyse the source medical image to determine, 505, the configuration of the MRI markers on the patient support device. That is, the positions of the markers and the inter marker distances can be determined. This information can be used to identify, 507, the patient support device. The computer may be configured to run a patient support device identification algorithm configured to identify a patient support device present in the source medical image based on the identified configuration of the plurality of intensity markers and the patient support device data. For example, the configuration of the MRI markers may be used to determine the dimensions and/or shape of the patient support device, which can be compared to information stored in the patient support device library to identify the imaged patient support device. The identified patient support device is then selected, for insertion into the modified medical image.

The reference plane is determined, based on the source medical image, by correlating the position of the patient support device in the frame of references coordinates (e.g., DICOM co-ordinates).

An sCT image is generated, 513, and a representation of the identified patient supported device is retrieved 515 and inserted 517 into the sCT.

This method can be used, in particular, to identify and insert patient support devices such as head rests and other treatment couch accessories into the image.

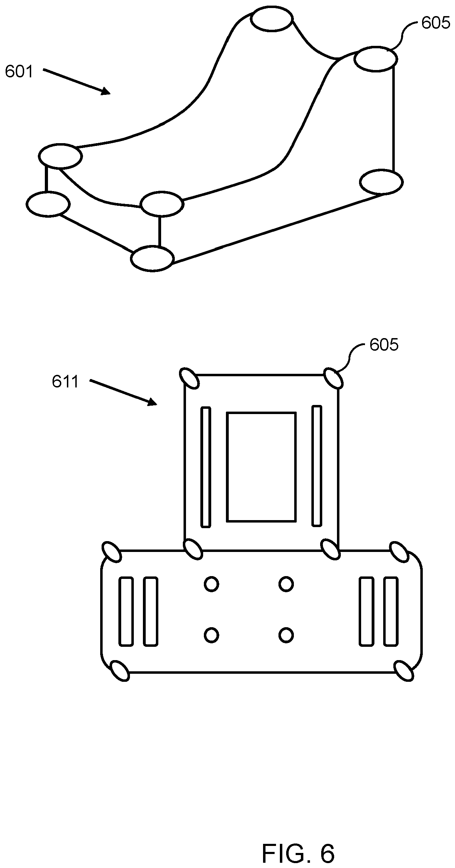

Fig 6 illustrates examples of patient support devices with MRI markers attached to them, for use in the method described in connection with Fig 5. In the uppermost image, the patient support device is a headrest 601. In the lower image, the patient treatment device is a base plate 611 for attaching the headrest to a treatment couch. The patient support devices have MRI markers 605 attached to them, to facilitate identification of the patient support device, as described in connection with Fig 5.

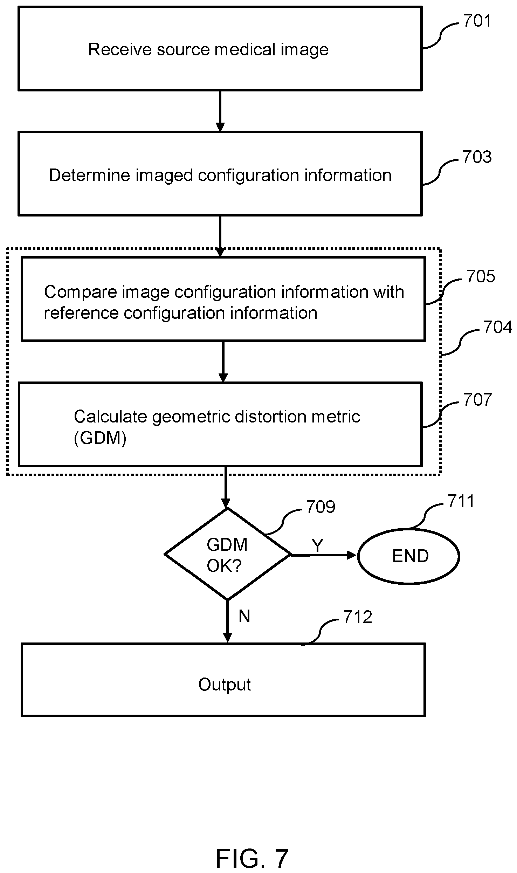

Fig 7 shows a flow diagram illustrating a computer implemented method for determining geometric fidelity of a medical image.

The method illustrated in Fig 7 can be used to determine the geometric fidelity of a source medical image e.g., an MRI image. The source medical image is obtained by scanning the patient on a patient support device, wherein the patient support device is marked with a plurality of markers e.g., MRI markers. When the MRI image is generated, using a reconstruction algorithm, the MRI markers appear in the image.

The method comprises receiving, 701, the source medical image and processing the image to detect the markers (via intensity detection). The positions of the markers in the image can be correlated with the image frame of reference (e.g., the DICOM frame of reference co-ordinates).

Imaged configuration information, i.e., information indicating the configuration of markers in the image is obtained, 703, from the image by analysing the image to determine the distances between the markers. The method may comprise determining the distances between the markers in the lateral and inferior-superior directions.

The method further comprises running a geometric distortion algorithm 704, which involves comparing, 705, the distances between the markers as determined from the image (i.e., based on the imaged configuration information) to the known actual distances between the markers on the patient support device.

Based on this information, the geometric distortion algorithm may determine, 707, geometric distortion information. Further, the method comprises outputting, 712, the geometric distortion information e.g., to a display, for viewing by a clinician.

In some embodiments, the geometric distortion information comprises calculated differences between the actual and imaged distances. In some embodiments, the geometric distortion information comprises a geometric distortion metric (GDM) indicating a measure of geometric distortion in the source medical image.

In some embodiments, the method further comprises generating an sCT image based on the source medical image and displaying the geometric distortion information during the step of generating the sCT image.

In some embodiments, the method further comprises analysing the geometric distortion information, 709, to determine if it is indicative of the level of geometric distortion in the source medical image being beyond an acceptable limit/tolerance level.

In response to determining that the level of geometric distortion in the source medical image is beyond an acceptable limit, the method may comprise generating an output indicating that the level of geometric distortion is unacceptably high and providing this information to a user (e.g., by display). In response to determining that the level of geometric distortion in the source medical image is within an acceptable limit, the method may terminate. Alternatively, the method may comprise generating an output indicating that the level of geometric distortion is acceptable and providing this information to the user (e.g., via a display device).

For example, the method may comprise comparing the geometric distortion metric to a threshold value and, in response to determining that the geometric distortion metric exceeds the threshold value, outputting a geometric distortion warning.

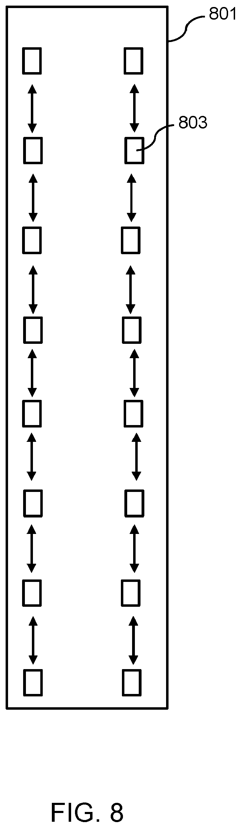

Fig 8 shows a schematic diagram of a patient support device 801 that is marked with a plurality of MRI markers 803. The patient support device 801 is a patient support couch, for example. The arrows indicate distances between the MRI markers, which can be measured and compared to the imaged distances to assess geometric distortion.

It will be appreciated that the method(s) described in connection with Fig 7, which are for assessing geometric distortion in a source medical image (e.g., an MRI image) can be combined with any of the disclosed methods of generating a modified medical image. This may assist accurate and efficient radiotherapy planning. The source medical image may first be assessed to determine the level of geometric distortion. If the level of geometric distortion is acceptable, the source medical image may be further processed to insert a representation of a patient support device and/or generate a sCT image, in accordance with various methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

It will be appreciated that while the methods of Fig 2 and Fig 4 are described in relation to a treatment couch, the same methods can be applied to other patient support devices (e.g., a head rest).

It will be appreciated that while the source medical image is described as being an MRI image in some examples, other types of medical image could be used. Further, while the modified medical image is described as being an sCT image in some examples, the modified medical image need not be an sCT image.

## Claims

1. A system for assisting radiotherapy planning, the system comprising:
a data storage comprising a patient support device library, the patient support device library comprising patient support device data for a plurality of patient support devices; and
a processor configured to:
receive a source medical image of a patient on a scanning couch;
determine a reference plane based on the source medical image;
select a patient support device from the patient support device library;
receive patient support device data for the selected patient support device; and
generate a modified medical image, based on the source medical image, wherein generating the modified medical image comprises positioning a representation of the selected patient support device relative to the reference plane.

2. The system of claim 1, wherein the source medical image is an MRI image obtained by magnetic resonance imaging and the processor is further configured to:
generate a synthetic computed tomography image based on the source medical image; and
generate the modified medical image by inserting a representation of the patient support device, at a location defined relative to the reference plane, into the synthetic computed tomography image.

3. The system of claim 1 or claim 2, wherein the patient support device data comprises:
(i) physical dimensions of the patient support device; and
(ii) an electron density property of the patient support device and/or a physical density property of the patient support device.

4. The system of any preceding claim, wherein the processor is further configured to:
receive a patient support device selection input; and
select the patient support device based on the patient support device selection input, wherein the patient support device selection input indicates a radiotherapy system for carrying out a planned radiotherapy treatment.

5. The system of any preceding claim, wherein the source medical image comprises a representation of a plurality of markers associated with the scanning couch and the processor is configured to determine the reference plane by:
detecting the plurality of markers associated with the scanning couch;
calculating the position of the scanning couch in the source medical image with respect to an image reference frame based on the locations of the plurality of markers; and
determining a reference plane based on the position of the scanning couch in the source medical image.

6. The system of any preceding claim wherein the patient support device data comprises information indicating a configuration of intensity markers characteristic of the patient support device and the processor is configured to:
process the source medical image to identify the configuration of a plurality of intensity markers;
run a patient support device identification algorithm configured to identify a patient support device present in the source medical image based on the identified configuration of the plurality of intensity markers and the patient support device data; and
determine the position of the patient support device in the source medical image relative to the reference plane based on the configuration of a plurality of intensity markers.

7. The system of any preceding claim wherein the patient support device comprises a treatment couch, a headrest and/or an accessory for fixing a headrest to a treatment couch.

8. The system of any preceding claim, wherein the source medical image comprises a representation of a plurality of markers and the processor is further configured to:
process the source medical image to obtain imaged configuration information indicating the configuration of the markers in the source medical image; and
run a geometric distortion algorithm, wherein the geometric distortion algorithm is configured to:
compare the imaged configuration information to reference configuration information, wherein the reference configuration information indicates the physical configuration of the markers; and
calculate and output geometric distortion information, based on the comparison of the imaged configuration information and the reference configuration information,
optionally wherein the geometric distortion information comprises a geometric distortion metric;
and further optionally wherein the imaged configuration information indicates the apparent distances between the markers based on the source medical image and the reference configuration information indicates the physical distances between the markers.

9. A method of radiation treatment planning, the method comprising:
obtaining a source medical image, using magnetic resonance imaging, wherein obtaining the source medical image comprises imaging a patient on a scanning couch;
processing the source medical image to obtain a synthetic computed tomography image;
determining a reference plane of the synthetic computed tomography image, based on the source medical image;
selecting a patient support device from a patient support device library;
receiving patient support device data; and
generating a modified medical image by inserting a representation of the patient support device, at a location defined relative to the reference plane, into the synthetic computed tomography image.

10. The method of claim 9 further comprising carrying out a dose calculation, based on the modified medical image.

11. A computer implemented method of processing a medical image, comprising:
receiving a source medical image of a patient on a scanning couch;
processing the source medical image to determine a reference plane;
selecting a patient support device from a patient support device;
receiving patient support device data for the selected patient support device; and
generating a modified medical image, based on the source medical image, by inserting a representation of the patient support device, at a location defined relative to the reference plane, into an image based on the source medical image.

12. The computer implemented method of claim 11 further comprising:
generating a synthetic computed tomography image based on the source medical image;
and
generating the modified medical image by inserting a representation of the patient support device into the synthetic computed tomography image.

13. The computer implemented method of claim 11 or claim 12, further comprising carrying out a dose calculation, based on the modified medical image.

14. The computer implemented method of any preceding claim, wherein the patient support device data comprises information indicating a configuration of intensity markers characteristic of the patient support device and the method further comprises:
identifying the configuration of a plurality of intensity markers present in the source medical image;
running a patient support device identification algorithm configured to identify a patient support device present in the source medical image based on the identified configuration of the plurality of intensity markers and the patient support device data; and
determining the position of the patient support device in the source medical image relative to the reference plane based on the configuration of a plurality of intensity markers.

15. A computer-readable storage medium comprising instructions which, when executed by a computer, cause the computer to carry out the method of any of claims 11-14.
